# DEMANDE DE BREVET EUROPEEN

(11) **EP 0 945 142 A1**
(43) Date de publication de la demande: **29.09.1999**
(21) Numéro de dépôt: 99400677.3
(22) Date de dépôt: 19.03.1999
(51) Int. Cl.: A61L 9/12, A61L 9/04

(54) **Dispositif désodoriseur à efficacité optimisée**

(30) Priorité: 24.03.1998 FR 9803607
(71) Demandeur: Société d'Electromenager du Nord Selnor, 59811 Lesquin (FR)
(72) Inventeur: Awtuch, Bernard, Thomson-CSF Propriété Intellect., 94117 Arcueil Cédex (FR); Charcosset, Henri, Thomson-CSF Propriété Intellec., 94117 Arcueil Cédex (FR)
(74) Mandataire: Chaverneff, Vladimir

(57) **Abrégé**

Le dispositif de l'invention comporte un bac (2) à deux récipients (4, 5) dont l'un contient du charbon actif (6) et l'autre un gel coloré auquel est incorporé de l'acide undécylénique (7) ou un de ses dérivés, le gel servant d'indicateur visuel de fin de période d'activité du charbon.

## Description

La présente invention se rapporte à un dispositif désodoriseur à efficacité optimisée.

Les absorbeurs d'odeurs comportent généralement un support poreux à base de charbon activé ou de zéolithe, auquel on ajoute des catalyseurs ou des agents oxydants et éventuellement germicides. Ces agents sont, par exemple, à base de métaux tels que le zinc, le cuivre, l'aluminium, Dans certains cas, on chauffe ces produits, ou on les excite aux rayons ultraviolets ou à l'ozone.

Par ailleurs, on a récemment découvert les bonnes propriétés d'absorption d'odeurs de l'acide undécylénique ou de ses dérivés, qui est commercialisé sous forme liquide ou sous forme d'aérosol ou de poudre (en mélange avec de la silice), et est généralement incorporé à la matière première à désodoriser.

Chacun de ces produits est efficace pour l'absorption d'odeurs d'origines différentes, et leurs spectres d'action respectifs ne sont pas très larges en général, et ne sont pas identiques.

Toutefois, les supports poreux posent le problème de leur saturation. Dans certaines applications, en particulier dans le domaine industriel, il est possible d'utiliser suffisamment de charbon actif pour couvrir une large période d'utilisation et/ou de le changer systématiquement à intervalles rapprochés, bien avant qu'il n'atteigne la saturation.

Par contre, dans d'autres domaines d'application, en particulier pour les réfrigérateurs, le volume disponible pour ces produits est limité, et les utilisateurs désirent bien entendu les utiliser le plus longtemps possible tout en obtenant une efficacité constante, ce qui est difficilement conciliable du fait qu'il est pratiquement impossible à un utilisateur ordinaire de déterminer à coup sûr et à l'avance le moment où le charbon actif sera saturé en produits adsorbés et ne remplira donc plus son rôle. Par conséquent, soit il remplacera prématurément les produits absorbants d'odeurs, soit il dépassera le moment où cette saturation sera atteinte, et même sans s'en apercevoir rapidement, car il se peut qu'à ce moment le réfrigérateur dans lequel ces produits sont disposés ne contiendra pas ou contiendra trop peu d'aliments dégageant des odeurs gênantes.

On connait d'après la demande européenne 0495 631 un dispositif rafraîchisseur d'air dont les principes actifs sont des gels parfumés à évaporation lente dont il est facile de voir le niveau. Dans le cas où les produits désodoriseurs sont à niveau constant (c'est à dire non évaporables), comme par exemple le charbon actif, ce document n'apporte pas de solutions au problème de la détermination de la fin d'efficacité de ces produits.

La présente invention a pour objet un désodoriseur qui permette de déterminer rapidement, facilement et même à l'avance le moment où ses qualités commenceront à se dégrader, ce dispositif étant peu onéreux, peu encombrant et ayant un spectre d'absorption d'odeurs le plus large possible, et qui soit efficace le plus longtemps possible.

Le dispositif désodoriseur à efficacité optimisée, conforme à l'invention est du type comprenant au moins un récipient dans lequel est disposé au moins un produit désodoriseur non évaporable, ce récipient étant parcouru par les gaz à désodoriser et il est caractérisé en ce qu'il comporte un indicateur visuel de fin d'efficacité d'au moins l'un des produits désodoriseurs non évaporables. De façon avantageuse, l'indicateur visuel est un gel coloré à évaporation lente, le temps d'évaporation du gel correspondant à la durée estimée d'activité du produit désodoriseur. Selon un mode de réalisation, le produit désodoriseur contient du charbon actif et il est disposé dans un récipient différent de celui dans lequel est disposé le gel. Le produit désodoriseur peut aussi être de l'acide undécylénique et il est alors incorporé au gel.

La présente invention sera mieux comprise à la lecture de la description détaillée d'un mode de réalisation, pris à titre d'exemple non limitatif et illustré par le dessin annexé, sur lequel :

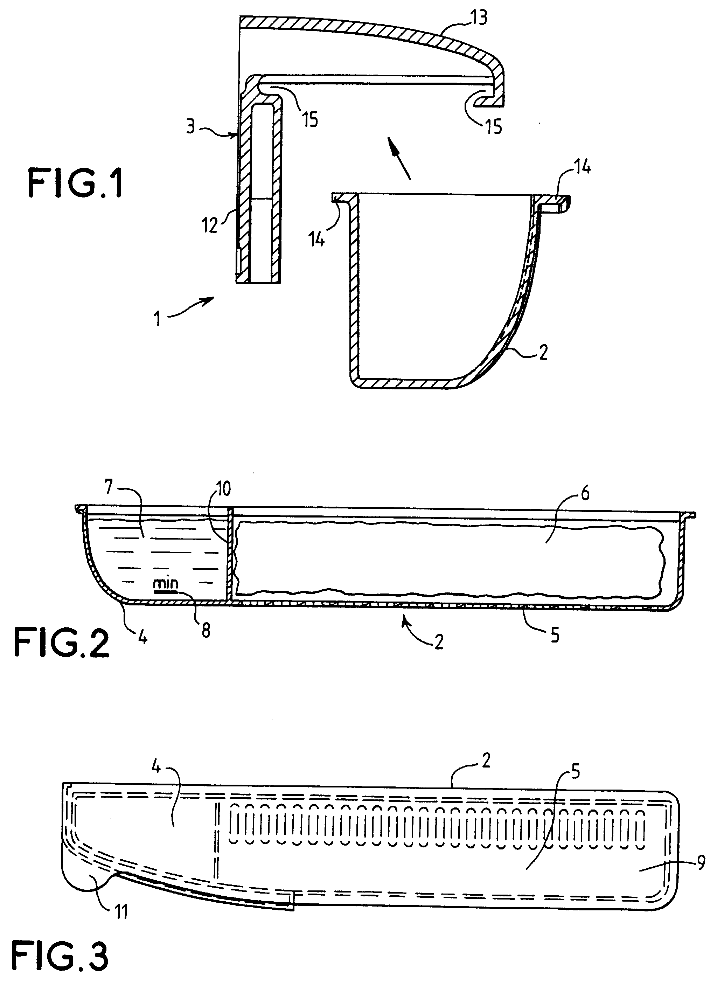

- la figure 1 est une vue en coupe transversale d'un dispositif désodoriseur conforme à l'invention,
- la figure 2 est une vue de face en coupe des récipients du dispositif de la figure 1, et
- la figure 3 est une vue de dessus du dispositif de la figure 2.

L'invention est décrite ci-dessous en référence à un dispositif désodoriseur pour réfrigérateur, mais il est bien entendu qu'elle n'est pas limitée à cette seule application, et qu'elle peut être mise en oeuvre dans d'autres appareils, dans des locaux et tous endroits nécessitant l'absorption d'odeurs, que ces odeurs aient une source unique ou plusieurs sources. Dans la présente description, on utilise le terme générique de « dispositif désodoriseur », ce qui veut dire que celui-ci peut agir par absorption de molécules du (ou des) gaz à désodoriser (par exemple par adsorption par du charbon actif), et/ou par dégradation (ou neutralisation) de ces molécules.

Le dispositif 1 représenté sur le dessin comporte un bac 2 et son support 3. Le bac 2 est en matière plastique, par exemple en polystyrène « cristal » (transparent) divisé en deux compartiments 4, 5. Le compartiment 5 a un volume intérieur environ trois fois plus grand que le volume du compartiment 4. Dans le compartiment 5, on dispose du produit à grande surface adsorbante, qui est, dans le cas présent, un produit poreux tel que du charbon actif 6. Ce charbon actif est enfermé dans une enveloppe, en matériau textile non tissé par exemple. Bien entendu, tout autre produit poreux et/ou granuleux ayant des propriétés similaires, ou un mélange de tels produits, peut remplacer le charbon actif. Ce produit peut par exemple être de la zéolithe, de l'alumine activée, de l'argile, du kieselguhr, ....

Dans le compartiment 4, on dispose une charge 7 de gel coloré auquel est incorporé de l'acide undécylénique (commercialisé sous la dénomination « C 11 ») ou un de ses dérivés. Ce gel est un gel à évaporation lente (par exemple une charge d'environ 30 à 40 cm³ doit pouvoir s'évaporer, dans des conditions normales d'utilisation en 2 à 4 mois environ. Ce gel est avantageusement un gel composé exclusivement d'ingrédients alimentaires, par exemple un mélange de farine de caroube (E410), de carraghénane kappa semi-raffiné (E407), d'un colorant bleu (tel que le sel calcique E131) et d'eau. Dans ce gel, on incorpore du « C11 » à proportion de 10 % environ. La quantité et la composition du produit mis dans le compartiment 4 sont déterminées une fois pour toutes de façon expérimentale pour que, lorsque l'efficacité du charbon actif (ou produit similaire) disposé dans le compartiment 5 descend en-dessous d'un seuil déterminé (par exemple lorsque le charbon est proche de la saturation), le niveau du gel dans le compartiment 4 soit nul, ou de préférence, égal à un minimum (marqué par un trait 8 et la mention « min » sur la paroi extérieure du compartiment 4) proche du zéro, afin d'être plus facilement repérable par l'utilisateur.

Le compartiment 5 est perforé à sa partie inférieure, et l'ensemble des deux compartiments 4, 5 est recouvert d'un opercule 9 soudé sur la face supérieure des parois du bac 2 et de la paroi intérieure 10 séparant les compartiments 4 et 5. L'opercule 9 comporte une languette 11 en saillie, qui permet à l'utilisateur de la détacher du bac 2 juste avant la mise en service du bac 2.

Le support 3 du bac 2 se compose essentiellement d'une paroi latérale 12 (dirigée verticalement lorsque le support est fixé dans le réfrigérateur) et d'un « chapeau » 13 destiné à recouvrir le bac 2. Pour permettre une fixation rapide du bac 2 sur le support 3, on forme à la partie supérieure des parois du bac un rebord périphérique 14 qui s'étend pratiquement perpendiculairement aux parois latérales du bac. L'opercule 9 est soudé sur ce rebord 14. Le rebord 14 s'engage en glissant dans des gorges latérales correspondantes 15 faisant office de glissières et formées sur la face inférieure du chapeau 13 du support 3. De façon avantageuse, ces gorges 15 peuvent comporter des petits renfoncements coopérant par encliquetage avec des petites protubérances formées sur les rebords 14 (non représentés), afin de bien verrouiller en position le bac 2 dans son support 3. Le support 3 est fixé par tout moyen approprié sur une paroi du réfrigérateur, par exemple par encliquetage. Le chapeau 13 comporte des perforations sur toute sa longueur, afin de permettre une bonne circulation de l'air à désodoriser à travers les deux compartiments 4, 5 du bac 2. Cette circulation d'air se fait par convection naturelle de l'air à l'intérieur du réfrigérateur. En variante, cette circulation peut être forcée, généralement de façon périodique.

Ainsi, grâce au dispositif de l'invention, on obtient non seulement une utilisation optimale des produits du bac 2, mais également une indication visuelle très commode de la fin de leur période d'activité (et l'on peut même prévoir suffisamment à l'avance cette fin d'activité) ainsi qu'un élargissement du spectre d'absorption d'odeurs de chacun des deux composants principaux dont les actions sont combinées (charbon actif ou produit similaire et « C 11 »).

Bien entendu, on peut associer aux produits précités à action désodorisante, un ou plusieurs produits à action bactériostatique, par exemple en en imprégnant l'enveloppe du charbon actif. Les deux récipients 4,5 décrits ci-dessus ont été représentés adjacents, côte à côte, mais il est bien entendu qu'ils peuvent être séparés, par exemple le récipient 4 disposé sous le récipient 5, ou à proximité de ce dernier.

## Revendications

1. Dispositif désodoriseur à efficacité optimisée, comprenant au moins un récipient (2) dans lequel est disposé au moins un produit désodoriseur (6, 7) non évaporable, ce récipient étant parcouru par les gaz à désodoriser, caractérisé en ce qu'il comporte un indicateur visuel (7, 8) de fin d'efficacité d'au moins l'un des produits désodoriseurs non évaporables.

2. Dispositif selon la revendication 1, caractérisé en ce que l'indicateur visuel est un gel coloré à évaporation lente, le temps d'évaporation du gel correspondant à la durée estimée d'activité du produit désodoriseur.

3. Dispositif selon la revendication 1 ou 2, caractérisé en ce que le produit désodoriseur contient du charbon actif (6) et qu'il est disposé dans un récipient (5) différent de celui (4) dans lequel est disposé le gel.

4. Dispositif selon l'une des revendications 2 ou 3, caractérisé en ce que le produit désodoriseur est de l'acide undécylénique ou un dérivé de cet acide, et qu'il est incorporé au gel.

5. Dispositif selon l'une des revendications 2 à 4, caractérisé en ce que qu'il comporte essentiellement deux produits désodoriseurs, dont l'une est à base de charbon actif, et l'autre de l'acide undécylénique, ou un dérivé de cet acide, incorporé au gel, et que le rapport de leurs volumes est d'environ 3 à 1 respectivement.

6. Dispositif selon l'une des revendications précédentes, caractérisé en ce qu'il comprend également des produits bactériostatiques.

7. Dispositif selon l'une des revendications précédentes, caractérisé en ce qu'il est disposé dans un réfrigérateur et comporte un support (3) fixé à une paroi du réfrigérateur, et un bac (2) à au moins un récipient (4, 5) pouvant être fixé de façon amovible sur le support (14, 15).

8. Dispositif selon l'une des revendications précédentes, caractérisé en ce qu'au moins le récipient contenant l'indicateur visuel est scellé hermétiquement à l'aide d'un opercule (9) détachable par l'utilisateur à sa mise en service.
